**Europäisches Patentamt**

**European Patent Office**

**Office européen des brevets**

(19)

(11) Veröffentlichungsnummer: **0 033 492**
**A1**

(12) **EUROPÄISCHE PATENTANMELDUNG**

(21) Anmeldenummer: 81100483.7

(22) Anmeldetag: 23.01.81

(51) Int. Cl.³: **A 61 C 5/10**
A 61 C 19/04, A 61 B 5/10
G 05 B 19/42

(30) Priorität: 31.01.80 DE 3003435

(43) Veröffentlichungstag der Anmeldung:
**12.08.81 Patentblatt 81/32**

(84) Benannte Vertragsstaaten:
**AT BE CH DE FR GB IT LI LU NL SE**

(71) Anmelder: **Becker Dental-Labor GmbH.**
**Rotter Bruch 24**
**D-5100 Aachen(DE)**

(72) Erfinder: **Becker, Günter**
**Rotter Bruch 24**
**D-5100 Aachen(DE)**

(72) Erfinder: **Weiden, Horst**
**Buttergasse 14**
**D-5190 Stolberg(DE)**

(74) Vertreter: **Selting, Günther, Dipl.-Ing. et al,**
**Deichmannhaus**
**D-5000 Köln 1(DE)**

(54) Verfahren und Vorrichtung zur Herstellung eines Kronenteiles.

(57) Zur Herstellung eines auf einem Zahnstumpf zu befestigenden Kronenteiles wird zunächst ein Gipsmodell des Zahnstumpfes hergestellt. Die Kontur dieses Gipsmodells wird mit einem Taststift (13) abgetastet und die Konturdaten werden in einen Speicher (48) eingegeben. Aus den Konturdaten wird in einem Rechner (50) die Kontur des herzustellenden Kronenteiles und der Materialzugabe errechnet. Auf das Gipsmodell des Zahnstumpfes wird ein Formmaterial aufgetragen und anschließend wird ein Werkzeug (16) entsprechend der vom Rechner (50) gelieferten Kontur geführt, um das Formmaterial zu bearbeiten.

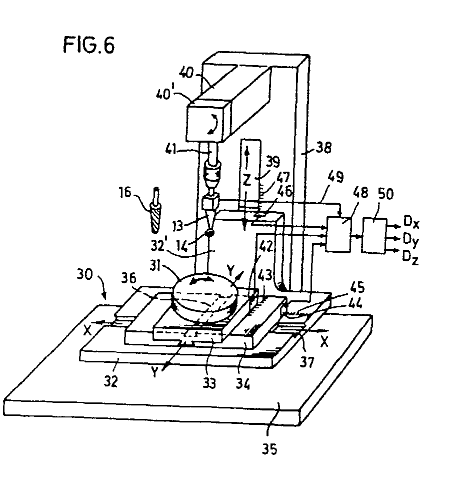

FIG.6

**VON KREISLER    SCHÖNWALD    EISHOLD    FUES**

**VON KREISLER    KELLER    SELTING    WERNER**

0033492

Anmelderin:

BECKER
DENTAL-LABOR GMBH.
Rotter Bruch 24
5100 AACHEN

PATENTANWÄLTE

Dr.-Ing. von Kreisler † 1973

Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

Sg/my

30.Januar 1980

DEICHMANNHAUS AM HAUPTBAHNHOF

**D-5000 KÖLN 1**

## Verfahren und Vorrichtung zur Herstellung eines Kronenteiles

Die Erfindung betrifft ein Verfahren zur Herstellung eines auf einem Zahnstumpf zu befestigenden Kronenteiles, bei welchem

a) ein Modell des Zahnstumpfes hergestellt wird,

b) auf das Modell ein Formmaterial aufgetragen wird,

c) die Außenkontur des Formmaterials durch Materialabtrag verformt wird, und

d) das Formmaterial von dem Modell entfernt und als Gießmodell für das Kronenteil benutzt wird, sowie eine Vorrichtung zur Durchführung des Verfahrens.

Bei der Herstellung von Kronen für den Zahnersatz wird normalerweise so vorgegangen, daß zunächst der Zahnarzt den zu ersetzenden Zahn abschleift, so daß ein Zahnstumpf erzeugt wird, an dem später das Kronenteil befestigt werden kann. Anschließend wird ein Gipsabdruck des gesamten Zahnkranzes einschließlich des abgeschliffenen Zahnstumpfes erzeugt und hieraus ein Gipsmodell des Zahnkranzes hergestellt. Das Gipsmodell wird auch als Sägemodell bezeichnet. Aus ihm können einzelne Zähne durch vertikale Sägeschnitte herausgetrennt werden, um separat bearbeitet zu werden. Auf das Gipsmodell des Zahnstumpfes wird Wachs als Formmaterial aufgetragen. Das Wachs wird entsprechend der Form des zu ersetzenden Zahnes mit einem Konturwerkzeug, z.B. einem Fräser, bearbeitet. Bei diesem Vorgang muß sichergestellt werden, daß auf allen Teilen der Umfangsfläche des Zahnstumpfes eine bestimmte Mindeststärke der Wachsschicht aufrechterhalten wird. Da der Zahntechniker beim Abfräsen des Wachses nicht sehen kann, wie stark die Wachsschicht an den einzelnen Stellen ist, erfordert dieser Vorgang ein Höchstmaß von Erfahrung und Feingefühl. Das so erzeugte Wachsmodell wird von dem Zahnstumpf abgenommen und als Gießmodell für das Kronenteil benutzt. Das Kronenteil wird aus einem hochwertigen Material, z.B. Gold, hergestellt.

Ein ähnlicher Vorgang findet bei der Herstellung von Teleskopkronen statt. Hierbei wird auf dem Zahnstumpf ein Primärteil, z.B. aus Gold, befestigt, das dem Zahnstumpf exakt angepaßt ist. Auf das Primärteil wird ein Sekundärteil aufgeschoben, das den künstlichen Zahn trägt und ebenfalls aus Gold bestehen kann.

Die Herstellung derartiger Kronenteile erfordert vom Zahntechniker nicht nur viel Erfahrung und außerordentlich genaue Arbeit, sondern es ergeben sich wegen des hochwertigen Kronenmaterials im Falle eines unzureichenden Arbeitsergebnisses auch hohe Materialkosten.

Aufgabe der Erfindung ist es, das Verfahren der eingangs genannten Art derart weiterzubilden, daß bei der Bearbeitung des auf das Modell des Zahnstumpfes aufgetragenen Formmaterials sichergestellt ist, daß alle Stellen der Oberfläche des Zahnstumpfes mit einer hinreichend starken Formmaterialschicht bedeckt sind.

Zur Lösung dieser Aufgabe ist erfindungsgemäß vorgesehen, daß

a1) die Kontur des Zahnstumpfes mindestens teilweise abgetastet und in geometrische Daten umgesetzt wird,

a2) die geometrischen Daten in einen Speicher eingegeben werden,

a3) aus den geometrischen Daten die Kontur des Gießmodells unter Materialzugabe auf der gesamten Außenfläche des Zahnstumpfes errechnet wird

c1) und ein Konturwerkzeug entsprechend der errechneten Kontur des Gießmodells an dem auf dem Abdruck des Zahnstumpfes angeordneten Formmaterial entlanggeführt wird.

Dadurch, daß die Kontur des Zahnstumpfes abgetastet und die Konturdaten gespeichert werden, bleiben die Konturdaten auch dann erhalten und zugänglich, wenn der Zahnstumpf mit einer Schicht aus Formmaterial, z.B. Wachs, beschichtet ist. Die Bearbeitung des Formmaterials erfolgt unter Berücksichtigung der Konturdaten des unter dem Formmaterial befindlichen Zahnstumpfes, so daß sichergestellt ist, daß die Formmaterialschicht an keiner Stelle zu weit abgetragen wird,bzw. zu dünn wird. Andererseits kann, z.B. bei der Herstellung des Primärteiles einer Sekundärkrone,auch sichergestellt werden, daß die Materialstärke des Gießmodelles - und damit auch die Materialstärke des nach dem Gießmodell herzustellenden Kronenteiles - nicht größer wird als unbedingt erforderlich ist. Das Kronenteil erhält somit eine optimale Wandstärke, die an allen Stellen der Fläche des Zahnstumpfes hinreichend stark, an keiner Stelle aber stärker als notwendig ist. Der sehr schwierige Vorgang der Formgebung des Gießmodelles (Wachsmodelles) wird dadurch wenigstens teilweise automatisiert. Durch die Automatisierung wird die Entstehung von Ausschußarbeit vermieden und darüber hinaus sichergestellt, daß für das Kronenteil nicht mehr Gold benutzt wird als unbedingt erforderlich ist.

Wenn bei der Herstellung des Primärteiles einer Teleskopkrone das Primärteil nach der Form des Gießmodelles gegossen worden ist, erfordert die Außenfläche des Primärteiles noch eine Feinbearbeitung bzw. Glättung, um Gießfehler zu beseitigen. Auch diese Feinbearbeitung kann unter Berücksichtigung der gespeicherten Formdaten des Zahnstumpfes dadurch erfolgen, daß das nach dem Gießmodell erzeugte Kronenteil auf das Modell des Zahnstumpfes aufgesetzt und entsprechend der errechneten Kontur des Gießmodelles, jedoch mit einem geringen Mindermaß, feinbearbeitet wird. Besonders vorteilhaft ist hierbei, daß das Kronenteil auf allen Stellen seiner Oberfläche einen konstanten Materialabtrag erfährt, wie er bei manueller Bearbeitung nicht erzielt werden kann. Dadurch werden Rauhigkeiten und Fehlstellen an der Oberfläche des Kronenteiles beseitigt und dennoch wird das Kronenteil exakt auf die Form des Zahnstumpfes abgestimmt, auf den es aufgesetzt werden soll.

Dies bedeutet jedoch nicht, daß die Außenfläche des Kronenteiles parallel zur Außenfläche des Zahnstumpfes verläuft. Wenn der Zahnstumpf schräg im Zahnkranz steht, werden seine Seitenflächen dennoch mit parallelen Kanten erzeugt, so daß der Zahnersatz durch paralleles Aufschieben relativ zu den übrigen Zähnen in den Mund eingepaßt und erforderlichenfalls auch wieder herausgenommen werden kann.

Eine Vorrichtung zur Durchführung des erfindungsgemäßen Verfahrens ist dadurch gekennzeichnet, daß eine numerisch gesteuerte Bearbeitungsmaschine mit einer Aufspannvorrichtung für das Modell einer Zahngruppe oder eines Zahnstumpfes ausgestattet ist und einen Tastkopf sowie eine Führungsvorrichtung zum Entlangführen des Tastkopfes an dem Zahnstumpf aufweist, daß der Tastkopf an einen Speicher angeschlossen ist, der beim Entlangführen des Tastkopfes an dem Zahnstumpf erzeugte Positionsdaten speichert, und daß anstelle des Tastkopfes oder zusätzlich zu diesem ein Konturwerkzeug vorgesehen ist, das in Abhängigkeit von dem Inhalt des Speichers unter Steuerung durch einen Rechner relativ zu dem Zahnstumpf bewegbar ist.

Bevor eine Bearbeitung erfolgt, wird zunächst der Tastkopf an dem Zahnstumpf entlanggeführt, um die Oberflächendaten des Zahnstumpfes in den Speicher einzugeben. Anschließend erfolgt das Auftragen des Formmaterials auf den Zahnstumpf und dann die automatische Bearbeitung des aufgetragenen Formmaterials. Während dieser Vorgänge bleibt das Modell des Zahnstumpfes unverrückbar fest in der Aufspannvorrichtung. Dies hat den Vorteil, daß der bei der Abtastung gewählte, bzw. von der Bearbeitungsmaschine vorgegebene Nullpunkt nicht justiert, bzw. in ein anderes Koordinatensystem umgerechnet werden muß.

Bei der Bearbeitungsmaschine müssen der Tastkopf und das Konturwerkzeug relativ zu dem Werkstück geführt bzw. bewegt werden können. Dabei ist es unerheblich, ob das Werkstück feststeht und der Tastkopf,bzw. das Konturwerkzeug bewegt wird oder ob das Werkstück (Modell) in einem räumlichen Koordinatsystem bewegt wird und der Tastkopf, bzw. das Konturwerkzeug, ortsfest angeordnet sind.

Zur Aufnahme der Konturdaten des Zahnstumpfes weist in zweckmäßiger Ausgestaltung der Erfindung der Tastkopf einen Berührungssensor auf, der nur dann die Einspeicherung von Positionsdaten in den Speicher zuläßt, wenn der Tastkopf ein anderes Teil berührt. Auf diese Weise wird sichergestellt, daß nur dann Positionsdaten in den Speicher eingegeben werden, wenn der Tastkopf mit dem abzutastenden Modell in Kontakt ist.

Im folgenden wird ein Ausführungsbeispiel der Erfindung unter Bezugnahme auf die Figuren näher erläutert.

Es zeigt:

Fig. 1 eine perspektivische Ansicht eines Gipsmodelles eines Unterkiefers mit einem geschliffenen Zahnstumpf,
Fig. 2 das Abtasten der Außenkontur des Zahnstumpfes,
Fig. 3 das Abfräsen einer auf den Zahnstumpf aufgebrachten Wachsschicht,
Fig. 4a,b und c verschiedene Phasen bei der Herstellung des Kronenteiles,
Fig. 5 ein Wachsmodell eines Kronenteiles über einem schrägstehenden Zahnstumpf, und
Fig. 6 eine schematische Darstellung einer Bearbeitungsmaschine zur Durchführung der Bearbeitungsvorgänge.

Bei der Herstellung eines Zahnersatzes wird ein in Fig. 1 dargestelltes Gipsmodell 10 des betreffenden Zahnkranzes in bekannter Weise hergestellt. Vor der Herstellung des Gipsmodelles wurde im Munde des Patienten ein Zah, der ersetzt werden soll, beschliffen, so daß ein Zahnstumpf entstanden ist. Das Modell des Zahnstumpfes ist in Fig. 1 mit 11 bezeichnet. An dem Gipsmodell 10 der Fig. 1 können einzelne Zähne durch vertikale Sägeschnitte 12 herausgetrennt werden, so daß sie separat bearbeitet werden können.

Das Gipsmodell 10 wird mit einer Aufspannvorrichtung an einer Bearbeitungsmaschine befestigt. Die Bearbeitungsmaschine weist einen Taststift 13 auf, an dessen Ende sich ein Tastkopf 14 befindet. Der Tastkopf 14 wird nun an der Außenfläche des Zahnstumpfes 11 entlangbewegt. Dies kann beispielsweise dadurch geschehen, daß der Tastkopf 14 nacheinander auf Kreisbahnen in unterschiedlichen Höhen um den Zahnstumpf 11 herumgeführt wird. Der Taststift 13 kann beispielsweise von Hand so bewegt werden, daß der Tastkopf 14 die Oberfläche des Zahnstumpfes 11 abtastet. Dabei wird über eine Koordinaten-Führungsvorrichtung jeweils die Position des Tastkopfes 14 ermittelt und in einen Speicher eingegeben. Am Ende der Abtastung enthält der Speicher die geometrischen Daten der Oberfläche des Zahnstumpfes 11.

Anschließend wird auf den Zahnstumpf 11 ein Formmaterial 15, beispielsweise Wachs , aufgetragen, so daß der Zahnstumpf 11 vollständig bedeckt ist.

Der Taststift 13 wird durch ein Konturwerkzeug, beispielsweise einen Fräser 16 ersetzt, der um seine Achse rotieren kann, und der von einem an den Speicher angeschlossenen Rechner derart geführt wird, daß die Wachsschicht 15 an allen Stellen eine konstante Stärke erhält. Das auf diese Weise hergestellte Wachsmodell 17 des Zahnstumpfes hat somit eine konstante Wandstärke. Dies wird dadurch erreicht, daß in dem Rechner die Konturdaten des Zahnstumpfes 11 in der Weise verarbeitet werden, daß eine Materialzugabe erfolgt, und daß anschließend der Fräser 16 von dem Rechner entlang der errechneten Außenkontur des Wachsmodelles 17 bewegt wird.

In Fig. 4 sind die verschiedenen Arbeitsschritte bei der Herstellung eines Kronenteiles 19 als Primärteil einer Teleskopkrone dargestellt. Der Zahnstumpf 11 wird zunächst abgetastet und seine Positionsdaten werden in einen Speicher eingegeben. Dann wird der Zahnstumpf 11 mit einer Wachsschicht umgeben, aus der das Wachsmodell 18 in der Weise geformt wird, daß das Wachsmodell eine umlaufende, senkrecht von dem Kiefer 20 ansteigende Außenwand 19 erhält und oberhalb des Zahnstumpfes 11 mit einer horizontalen Stirnfläche 21 endet. Diese Formgebung bewirkt, daß das nach dem Wachsmodell 18 herzustellende Kronenteil senkrecht von dem Kiefer 20 abgehoben werden kann, und zwar auch dann, wenn dieses Kronenteil mit mehreren künstlichen Zähnen in Form einer Brücke verbunden ist.

Beim Fräsen des Wachsmodelles 18 ist es wichtig darauf zu achten, daß eine bestimmte Mindeststärke a der Wachsschicht nicht unterschritten wird. Die andere Bedingung besteht darin, daß die Seitenwände 19 senkrecht bzw. leicht konisch auftragen müssen.

Entsprechend dem Wachsmodell 18 wird anschließend das Kronenteil 19 gegossen und auf das Modell des Zahnstumpfes 11 gemäß Fig. 4c aufgesetzt. Das Kronenteil 19, das aus Gold besteht, wird entlang seiner Außenfläche nachgefräst, wobei ein Materialabtrag von beispielsweise 1/10 mm erfolgt. Diese Feinbearbeitung erfolgt ebenfalls durch die numerisch gesteuerte Bearbeitungsmaschine, die ja die Außenkontur des von ihr durch Fräsen hergestellten Wachsmodelles 18 gespeichert enthält, und die Feinbearbeitung lediglich mit einem geringen Mindermaß 21 durchführen muß.

Das in Fig. 5 dargestellte Gipsmodell 22 eines Zahnstumpfes ist schräg zur Seite geneigt. Das Wachsmodell 23 des herzustellenden Kronenteiles erhält auch hier parallele vertikale Wände 24, wobei durch Berechnung der Wandstärke dafür gesorgt ist, daß die Mindeststärke a in allen Bereichen einen festgelegten Wert nicht unterschreitet. Der Rechner nimmt also die Ausrechnung des Verlaufs der Seitenwände 24 in der Weise vor, daß die Mindeststärke a an keiner Stelle unterschritten wird.

In Fig. 6 ist eine Bearbeitungsmaschine 30 dargestellt, die eine Aufspannvorrichtung 31 aufweist, auf der das Zahnmodell 10 (Fig. 1) befestigt werden kann. Die Aufspannvorrichtung 31 ist um eine vertikale Achse drehbar auf einem Quersupport 33 befestigt, der in Richtung der Y-Achse auf einem Längssupport 34 bewegt werden kann. Der Längssupport 34 kann in Richtung der X-Achse auf einer Basisplatte 35 verfahren werden. Der Quersupport 33 ist in einer Führungsschiene 36, die quer auf dem Längssupport 34 verläuft, geführt, während der Längssupport 34 in einer Führungsschiene 37 einer vertikal verfahrbaren Hubplatte 32 geführt ist.

Der Längssupport 34 und der Quersupport 33 sind mit (nicht dargestellten) Antriebseinrichtungen versehen, mit denen sie entlang der Führungsschienen 37 bzw.36 gesteuert bewegt werden können.

An der Basisplatte 35 ist ein aufragender Ständer 38 befestigt, der eine vertikale Führungsschiene 39 aufweist, in der ein vertikales Führungsteil 32' der Hubplatte 32 geführt ist. Der Ständer 38 trägt einen frei vorstehenden Balken 40, von dem eine rotatorisch antreibbare Spindel 41 in Richtung auf die Aufspannvorrichtung 31 frei nach unten ragt. An der Spindel 41 ist der Taststift 13 befestigt.

Der Quersupport 33 weist einen Positionsgeber 42 auf, der Strichmarkierungen 43, die in Richtung der Y-Achse auf dem Längssupport 34 angebracht sind, abtastet und auf diese Weise die Position des Quersupports in Richtung der Y-Achse in numerischen Daten ermittelt.

In gleicher Weise weist der Längsupport 34 einen Positionsgeber 44 auf, der auf Positionsmarkierungen 45 anspricht, die parallel zu den Führungsschienen 37 an der Basisplatte 35 angebracht sind. Der Positionsgeber 44 ermittelt auf diese Weise die Position des Längssupports 34 in Längsrichtung.

Ein dritter Positionsgeber 46 ist an dem Führungsteil 32' angebracht. Der Positionsgeber 46 reagiert auf Positionsmarkierungen 47, die parallel zu der Führungsschiene 39 längs des Ständers 38 angebracht sind und ermittelt die Position der Hubplatte 40 und damit auch die Position des Tastkopfes des Taststiftes 13 in Z-Richtung.

Die Positionsdaten aller drei Positionsgeber 42, 44 und 46 werden einem elektronischen Speicher 48 zugeführt. Die Eingabe in den Speicher erfolgt nur dann, wenn an dem Tastkopf eine Berührung mit einem anderen Gegenstand festgestellt wird. Dies wird dem Speicher 48 über eine Steuerleitung 49 mitgeteilt.

Ist das Gipsmodell 10 auf der Aufspannvorrichtung 31 befestigt, dann wird durch geeignete z.B. manuelle Bewegungen des Längssupports 34, des Quersupports 33 und der Hubplatte 32 dafür gesorgt, daß der Tastkopf 14 an der Oberfläche des Zahnstumpfes 11 entlangbewegt wird.Auf diese Weise werden für jeden Berührungspunkt bzw. jeden Oberflächenpunkt des Zahnstumpfes die X-Y und Z Daten in den Speicher 48 eingeschrieben. Im Speicher 48 entsteht aufgrund dieser Positionsdaten ein Bild der Außenfläche des Zahnstumpfes.

Der Speicher 48 ist an einen Rechner 50 angeschlossen, der aus der ermittelten Außenkontur des Zahnstumpfes die Außenkontur des herzustellenden Wachsmodelles errechnet, wobei er die vorgegebenen Randbedingungen berücksichtigt, beispielsweise eine Mindestmaterialstärke a bzw. die Tatsache, daß die Seitenwände des Wachsmodells senkrecht oder konisch sein sollen.
Nun wird der Taststift 13 durch den Fräser 16 ersetzt, der an der Spindel 41 angebracht wird, und die Spindel 41 wird in Drehung versetzt. Der Rechner 50 gibt Positionswerte $D_x$ für die Stellung des Quersupports 33, $D_y$ für die Stellung des Längssupports 34 und $D_z$ für die Vertikalposition der Hubplatte 32 aus. Diese Positionsdaten ändern sich in der Weise, daß der Fräser 16 entlang der gewünschten Kontur des Wachsmodelles bewegt wird, bis das Wachsmodell die von dem Rechner 50 ermittelte Außenkontur angenommen hat.

Zur Positionsregelung werden die Signale der Positionsgeber 42,44 und 46 als Rückkopplungssignale dem Regler 50 ebenfalls zugeführt.

Prinzipiell genügen zur numerischen Festlegung der Außenkontur eines Werkstückes die drei Koordinatenwerte X,Y und Z. Um den Tastkopf 14 und den Fräser 16 auch an schwer zugängliche Stellen heranführen zu können, kann das vordere Ende 40' des Balkens 40 um die horizontale Längsachse des Balkens herum schwenkbar sein, so daß die Spindel 40 aus ihrer vertikalen Grundstellung herausgeschwenkt werden kann. Außerdem ist es möglich, die Aufspannvorrichtung 31 um eine vertikale Achse drehbar zu lagern. Die Dreh- bzw. Schwenkpositionen der Teile 40' und 31 können ebenfalls durch Positionsfühler erfaßt und dem Speicher 48 bzw. dem Rechner 50 zugeführt werden.

ANSPRÜCHE

1. Verfahren zur Herstellung eines auf einem Zahnstumpf zu befestigenden Kronenteiles, bei welchem

   a) ein Modell des Zahnstumpfes hergestellt wird,
   b) auf das Modell ein Formmaterial aufgetragen wird,
   c) die Außenkontur des Formmaterials durch Materialabtrag verformt wird und
   d) das Formmaterial von dem Modell entfernt und als
      Gießmodell für das Kronenteil benutzt wird,

d a d u r c h     g e k e n n z e i c h n e t,    daß

   a1) die Kontur des Zahnstumpfes mindestens teilweise
       abgetastet und in geometrische Daten umgesetzt wird,
   a2) die gemometrischen Daten in einen Speicher eingegeben werden,
   a3) aus den geometrischen Daten die Kontur des Gießmodells unter Materialzugabe auf der gesamten Außenfläche des Zahnstumpfes errechnet wird,
   c1) und daß ein Konturwerkzeug entsprechend der errechneten Kontur des Gießmodelles an dem auf dem Abdruck
       des Zahnstumpfes angeordneten Formmaterial entlanggeführt wird.

2. Verfahren nach Anspruch 1, d a d u r c h     g e k e n n -
z e i c h n e t,    daß

   e) das nach dem Gießmodell erzeugte Kronenteil auf das
      Modell des Zahnstumpfes aufgesetzt und entsprechend
      der errechneten Kontur des Gießmodelles, jedoch mit
      einem geringen Mindermaß, feinbearbeitet wird.

3. Vorrichtung zur Durchführung des Verfahrens nach
Anspruch 1 oder 2,  d a d u r c h   g e k e n n -
z e i c h n e t,   daß eine numerisch gesteuerte Bearbeitungsmaschine (30) mit einer Aufspannvorrichtung (31)
für das Modell (10) einer Zahngruppe oder eines Zahnstumpfes ausgestattet ist und einen Tastkopf (14) sowie
eine Führungsvorrichtung zum Entlangführen des Tastkopfes
an dem Zahnstumpf aufweist, daß der Tastkopf an einen
Speicher (48) angeschlossen ist, der beim Entlangführen
des Tastkopfes an dem Zahnstumpf erzeugte Positionsdaten
(x,y,z) speichert und daß anstelle des Tastkopfes (14)
oder zusätzlich zu diesem ein Konturwerkzeug (16) vorgesehen ist, das in Abhängigkeit von dem Inhalt des Speichers (48) unter Steuerung durch einen Rechner (50) relativ zu dem Zahnstumpf (11) bewegbar ist.

4. Vorrichtung nach Anspruch 3,  d a d u r c h   g e -
k e n n z e i c h n e t,   daß der Tastkopf (14) einen Berührungssensor aufweist, der nur dann die Einspeicherung
von Positionsdaten (x,y,z) in den Speicher (48) zuläßt,
wenn der Tastkopf ein anderes Teil berührt.

5. Vorrichtung zur Herstellung von Kopien eines Original-
Gegenstandes insbesondere zur Durchführung des Verfahrens
nach Anspruch 1 oder 2,  d a d u r c h   g e k e n n -
z e i c h n e t, daß eine numerisch gesteuerte Bearbeitungsmaschine (30) an ein elektronisches Steuergerät angeschlossen ist, das einen Speicher (48) aufweist, in den während
einer  Abtastphase die Positionsdaten der Oberfläche
des Original-Gegenstandes eingelesen werden, und aus dem sie
während einer nachfolgenden Wiedergabephase ausgelesen werden, wobei ein Bearbeitungswerkzeug entsprechend der Oberfläche des Original-Gegenstandes an einem Rohling entlanggeführt wird.

FIG.1

10

11

12

FIG.2

13

11

14

FIG.3

16

17

15

11

11

20

FIG.4a

18

21

19

11

20

a

FIG.4b

a

22

23

24

a

FIG.5

11

19

21

20

FIG.4c

FIG.6

**Europäisches Patentamt**

## EUROPÄISCHER RECHERCHENBERICHT

EP 81 10 0483.7

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 4 182 312 (MUSHABAC) <br> * ganzes Dokument * <br> -- | 1,3-5 |
|  | US - A - 3 861 044 (SWINSON, JR.) <br> * Spalte 1, Zeile 8 bis Spalte 2, Zeile 19 * <br> -- | 1 |
|  | US - A - 3 624 371 (NEAL et al.) <br> * Ansprüche 1 und 2 * <br> -- | 3,5 |
|  | US - A - 3 600 660 (FORD MOTOR CO.) <br> * Spalte 1, Zeile 9 bis Spalte 2, Zeile 3 * <br> -- | 3,5 |
| A | DE - A - 2 048 791 (TAUCHMANN) <br> ---- | |

**KLASSIFIKATION DER ANMELDUNG (Int. Cl.³)**

A 61 C 5/10
A 61 C 19/04
A 61 B 5/10
G 05 B 19/42

**RECHERCHIERTE SACHGEBIETE (Int. Cl.³)**

A 61 B 5/00
A 61 C 5/00
A 61 C 9/00
A 61 C 13/00
A 61 C 19/00
G 05 B 19/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 23-04-1981 | SIMON |

EPA form 1503.1 06.78